# EUROPEAN PATENT SPECIFICATION

(11) **EP 0 870 515 B1**
(45) Date of publication and mention of the grant of the patent: **15.06.2005**
(21) Application number: 98106333.2
(22) Date of filing: 07.04.1998
(51) Int. Cl.: A61M 1/36

(54) **Blood reservoir, storing and delivery member, drive unit**
Blutbehälter, Aufbewahrungs- und Abgabe-Vorrichtung , und Antriebseinheit
Dispositif de stockage et de distribution de sang, et systeme d'entrainement

(30) Priority: 08.04.1997 JP 10673097; 01.12.1997 JP 34701297
(43) Date of publication of application: 14.10.1998
(73) Proprietor: TERUMO KABUSHIKI KAISHA, Tokyo (JP)
(72) Inventor: Kato, Yukitoshi, Ashigarakami-gun, Kanagawa-ken (JP); Maruyama, Shinji, Ashigarakami-gun, Kanagawa-ken (JP); Ogihara, Mitsuaki, Ashigarakami-gun, Kanagawa-ken (JP); Takeuchi, Kazuhiko, Ashigarakami-gun, Kanagawa-ken (JP)
(74) Representative: Casalonga, Axel

(56) References cited:
- EP-A- 0 390 380
- EP-A- 0 498 740
- EP-A- 0 587 251
- EP-A- 0 766 974
- US-A- 4 599 093
- US-A- 4 610 656

## Description

### BACKGROUND OF THE INVENTION

The present invention relates to a delivery blood storing member-equipped blood reservoir tank that is equipped with a delivery blood storing member for temporarily. storing blood and delivering stored blood in an extracorporeal blood circulation circuit for use with, for example, an artificial lung or the like. The invention also relates to a blood delivery instrument for an extracorporeal blood circulation circuit provided downstream from the blood reservoir tank.

Heart surgery often uses an extracorporeal blood circulation circuit incorporating an artificial lung for removing carbon dioxide from blood and adding oxygen to blood instead of the living lung. The extracorporeal circulation circuit drains blood from the patient's vein, subjects the blood to gas exchange in the artificial lung, and returns the blood to the patient's artery. The extracorporeal circulation circuit also includes a cardiotomy line for drawing blood from the operation field, removing foreign matter therefrom, and returning the blood.

Normally, the extracorporeal blood circulation circuit with an artificial lung includes a blood tank for temporarily storing blood drained from the patient and a cardiotomy reservoir for filtering blood sucked from the operation field and temporarily storing the blood. The blood tank and the cardiotomy reservoir serve a buffering function of adjusting the amount of blood in the circuit and maintaining a constant flow of blood returning to the patient.

If the volume of blood in the blood reservoir tank becomes zero during extracorporeal blood circulation, air will be fed into the extracorporeal circulation circuit by a blood feed pump in operation. To avoid such inconvenience, the blood reservoir tank is provided with a level sensor for monitoring the volume of blood in the tank. When the sensor detects that the volume of blood in the tank is equal to or less than a predetermined value, the blood delivery action of the feed pump is stopped until a certain volume of blood in the blood tank is recovered. The temporary interruption of blood delivery, however, is undesirable because it causes blood stagnation throughout the circuit including the artificial lung.

EP 0 766 974 A2 discloses a delivery blood storing member-equipped blood reservoir tank comprising a blood reservoir tank portion and a delivery blood storing member including a body part formed from hard material, and a flexible diaphragm whose peripheral end is fixed to the body part and which produces substantially no self-restoring force against deformation.

Therefore, there is a need to provide a delivery blood storing member-equipped blood reservoir tank capable of changing the flow of blood delivery in accordance with the amount of blood stored if the amount of blood stored in the blood reservoir tank becomes equal to or less than a predetermined value, and a blood delivery instrument for an extracorporeal blood circulation circuit disposed downstream from the blood reservoir tank.

It is an object of the invention to provide a blood delivery mechanism-equipped blood reservoir tank that is equipped with a blood delivery driving unit for pressing a delivery blood reserving member, wherein even if the blood delivery driving unit is designed to press the delivery blood storing member by expansion of a blood reserving member-pressurizing portion formed of an expandable sheet material, the blood reserving member-pressurizing portion does not affect the delivery blood reserving member when the blood reserving member-pressurizing portion is not expanded, so that when the amount of blood in a blood storing portion becomes equal to or less than a predetermined value, the delivery blood reserving member reserves amounts of blood in sensitive correspondence to the amount of blood stored.

Lately, the conveyance of blood into the blood reservoir tank and the drainage of blood from a patient are often performed by a method using a suction device (negative pressure applying device) connected to the blood reservoir tank. When this device is operated, the pressure in the blood reservoir tank portion becomes negative, and the negative pressure affects the interior of the delivery blood reserving member. That is, the delivery blood reserving member becomes exposed to a negative pressure inside the member due to the operation of the suction device (negative pressure applying device), while receiving the atmospheric pressure on the outside. Therefore, the delivery blood reserving member is pressed by a differential pressure between the inside and outside pressures, so that the liquid surface-sensitive (pressure-sensitive, pre-load-sensitive) function of the delivery blood reserving member may be impeded.

It is a further object of the invention to provide a blood delivery mechanism-equipped blood reservoir tank that will not impede the liquid surface-sensitive (pressure-sensitive, pre-load-sensitive) function of a delivery blood reserving member even if a suction device (negative pressure applying device) is connected to the blood reservoir tank.

### SUMMARY OF THE INVENTION

Subject of the invention is a blood reservoir tank with a blood delivery mechanism having the features of claims 1 and 3. An advantageous embodiment of the blood reservoir tank is indicated in claim 2.

As claimed, a blood reservoir tank equipped with a blood delivery mechanism is provided that includes a blood storing portion, and a blood reserving member including a blood reserving portion that communicates with the blood storing portion. The blood reserving portion reserves an amount of blood proportional to an amount of blood stored in the blood storing portion when the amount of blood stored in the blood storing portion becomes equal to or less than a predetermined value. The blood reserving portion allows blood to flow out of the interior thereof when pressed from outside. The blood delivery mechanism further includes a blood delivering drive unit that operates when blood is to be sent out of an interior of the blood reserving member. The blood reserving member includes a body portion of the blood reserving member which body portion is formed from a hard material, and a flexible diaphragm whose peripheral portion is retained to the body portion of the blood reserving member. The blood delivering drive unit includes a blood reserving member-pressurizing portion formed from an expandable sheet material, and a blood delivering fluid flow port for expanding and contracting the blood reserving member-pressurizing portion. The blood delivery mechanism-equipped blood reservoir tank further includes a communication passage that connects a space formed between the diaphragm and the blood reserving member-pressurizing portion to an upper portion of the blood storing portion.

Furthermore, as claimed a blood delivery mechanism-equipped blood reservoir tank is provided that includes a blood storing portion, and a blood reserving member including a blood reserving portion that communicates with the blood storing portion. The blood reserving portion reserves an amount of blood proportional to an amount of blood stored in the blood storing portion when the amount of blood stored in the blood storing portion becomes equal to or less than a predetermined value. The blood reserving portion allows blood to flow out of an interior thereof when pressed from outside. The blood delivery mechanism-equipped blood reservoir tank further includes a blood reserving member-pressurizing portion that operates when blood is to be sent out of the blood reserving member. The blood reserving member is a soft material-made tubular body. The blood reserving member-pressurizing portion is a pump that compresses the soft material-made tubular body to force blood out of an interior of the tubular body. The blood delivery mechanism-equipped blood reservoir tank further includes a case member separating the soft material-made tubular body and a pump site that is a portion that compresses the soft material-made tubular body to force blood out of the interior of the tubular body, air-tightly from the outside. A communication passage connects between an interior of the case member and an upper portion of the blood storing portion.

### BRIEF DESCRIPTION OF THE DRAWINGS

The foregoing and further objects, features and advantages of the present invention will become apparent from the following description of various embodiments with reference to the accompanying drawings, wherein like numerals are used to represent like elements and wherein:
Fig. 1 is a front elevation of a blood delivery mechanism-equipped blood reservoir tank with an artificial lung connected - which embodiment, however, does not belong to the invention;
Fig. 2 is a side view of the blood delivery mechanism-equipped blood reservoir tank, with an artificial lung connected;
Fig. 3 is a front elevation of the blood delivery mechanism-equipped blood reservoir tank shown in Fig. 1, with a fragmentary sectional view of a blood delivery mechanism and a lower portion of a blood storing portion and their surroundings;
Fig..4 is an enlarged sectional view of a blood delivery mechanism and a lower portion of the blood storing portion and their surroundings in the blood delivery mechanism-equipped blood reservoir tank shown in Fig. 1;
Fig..5 is an enlarged sectional view of the blood delivery mechanism and a lower portion of the blood storing portion and their surroundings in the blood delivery mechanism-equipped blood reservoir tank shown in Fig. 1, at the time of blood delivery;

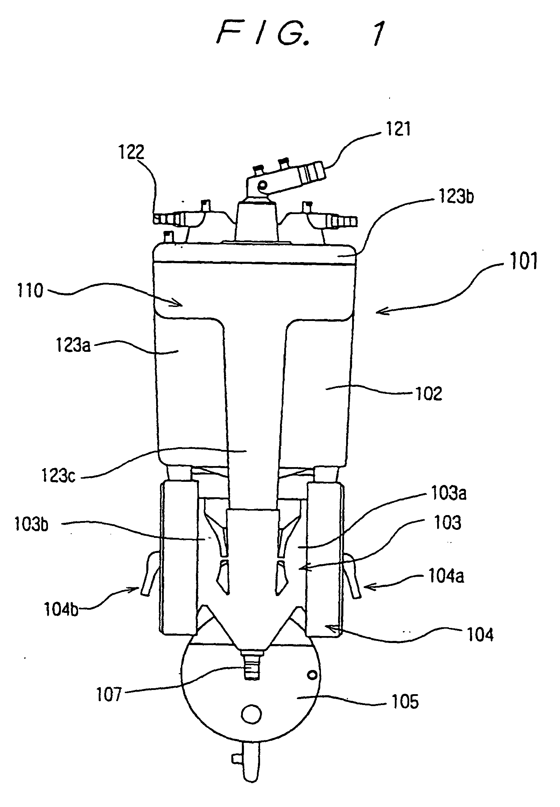

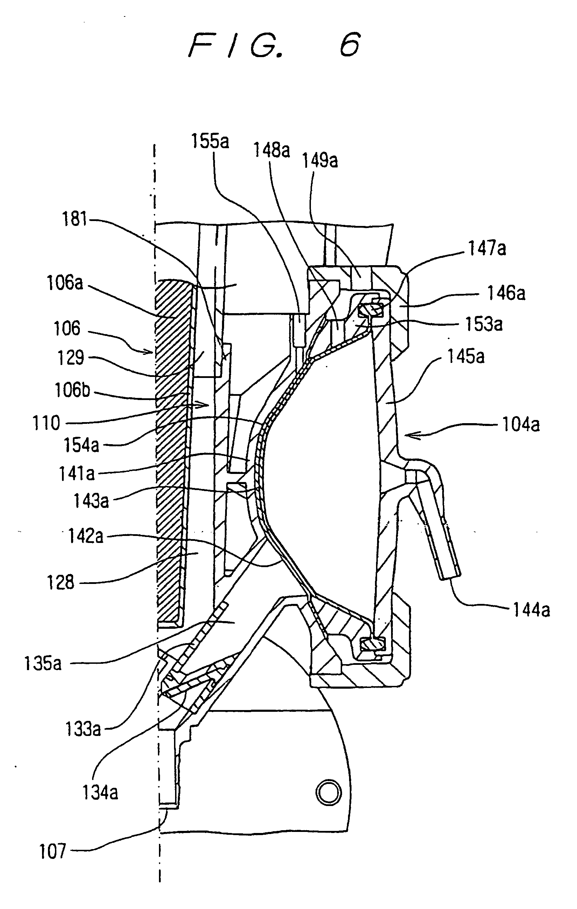

Fig. 6 is an enlarged sectional view of the blood delivery mechanism in the blood delivery mechanism-equipped blood reservoir tank shown in Fig. 1, at the time of blood delivery;

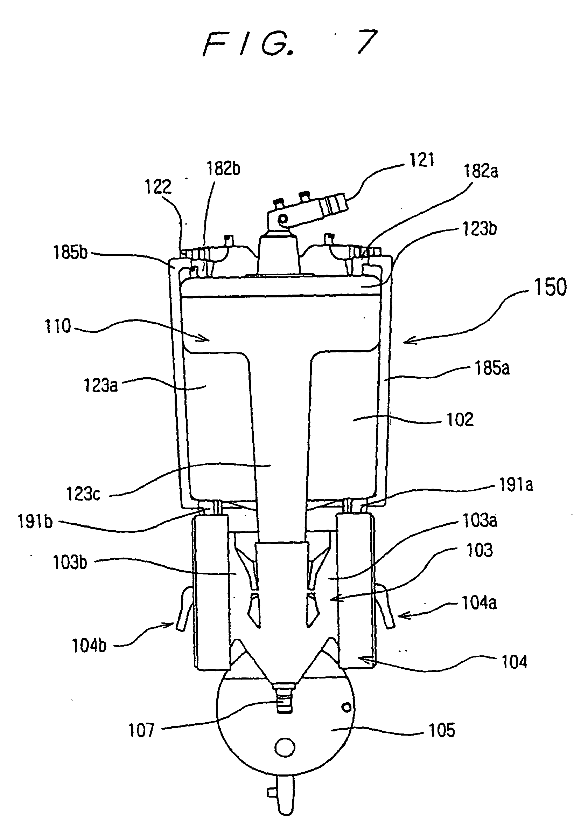

Fig. 7 is a front elevation of a blood delivery mechanism-equipped blood reservoir tank according to an embodiment of the present invention, with an artificial lung connected;
Fig..8 is a side view of the blood delivery mechanism-equipped blood reservoir tank according to the embodiment, with the artificial lung connected;
Fig..9 is an enlarged sectional view of the blood delivery mechanism and a lower portion of a blood storing portion and their surroundings in the blood delivery mechanism-equipped blood reservoir tank shown in Fig. 7, at the time of blood delivery;

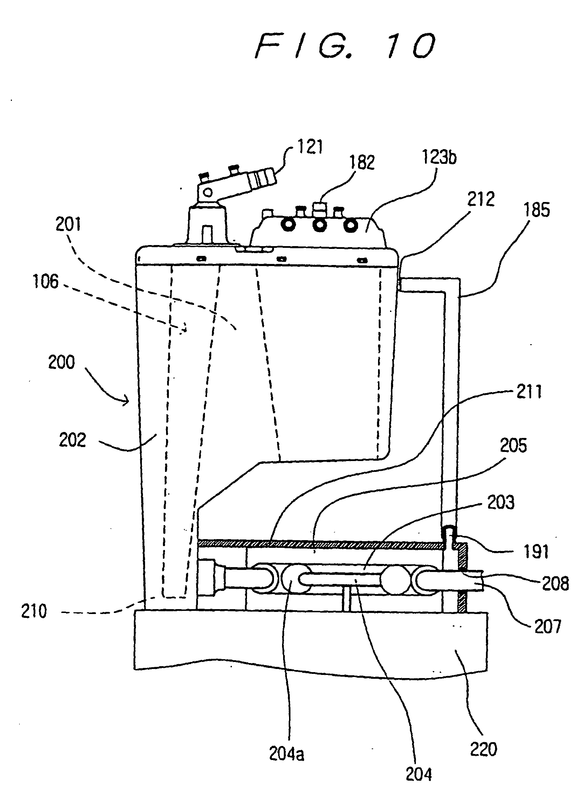

Fig. 10 is a partially cut-away side view of a blood delivery mechanism-equipped blood reservoir tank according a further embodiment of the present invention; and

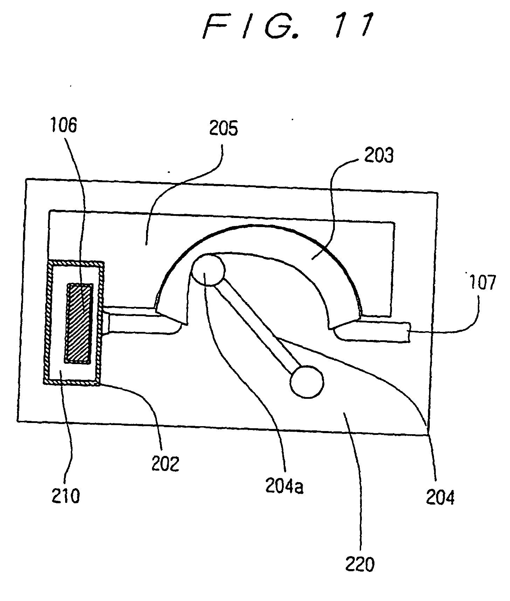

Fig. 11 is a sectional view of a lower portion of the blood delivery mechanism-equipped blood reservoir tank shown in Fig. 10, taken on a horizontal plane, wherein a case member is removed.

### DETAILED DESCRIPTION OF VARIOUS EMBODIMENTS

Embodiments of the delivery blood storing member-equipped blood reservoir tank of the present invention will be described in detail hereinafter with reference to the accompanying drawings.

Referring first to Figs. 1 through 6, a blood delivery mechanism-equipped blood reservoir tank is described that does not belong to the invention, however, yields a good understanding of the embodiments of the invention that are described later.

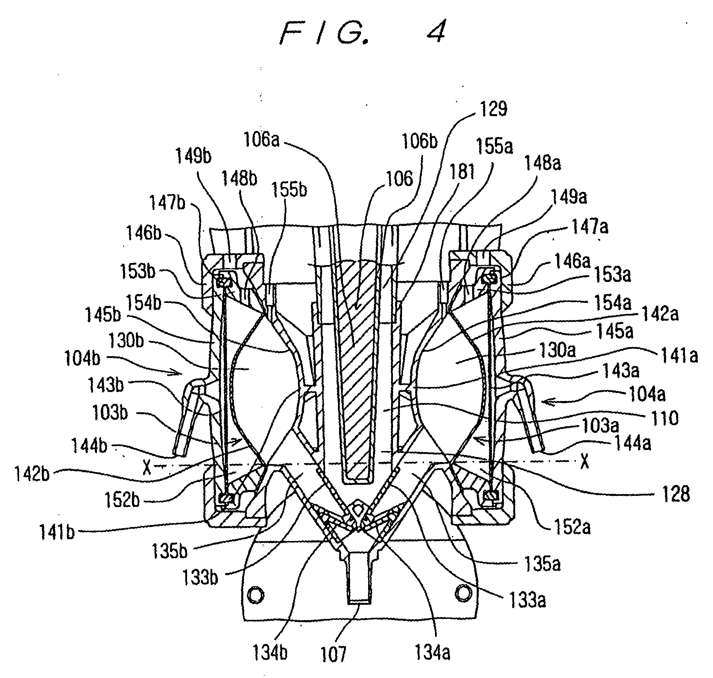

A blood delivery mechanism-equipped blood reservoir tank 100 according to an embodiment of the invention includes a blood storing portion 110, and blood reserving members 103a, 103b including blood reserving portions 130a, 130b that communicate with the blood storing portion 110. The blood reserving portions 130a, 130b reserve amounts of blood proportional to the amount of blood stored in the blood storing portion 110 when the amount of blood stored in the blood storing portion 110 becomes equal to or less than a predetermined value. The blood reserving portions 130a, 130b allow blood to flow out of the interiors thereof when pressed from outside. The blood delivery mechanism-equipped blood reservoir tank 100 further includes a bubble eliminating member 106 or a bubble removing member that is positioned inside the blood storing portion 110. A lower end of the bubble eliminating member 106 or the bubble removing member is positioned below lower ends of the blood reserving portions 130a, 130b of the blood reserving members 103a, 103b, that is, below an imaginary horizontal line X (Fig. 4) extending from the lower ends of the blood reserving portions 130a, 130b.

In other words, the bubble eliminating member 106 or the bubble removing member in the blood delivery mechanism-equipped blood reservoir tank 100 is provided so that the lower end of the bubble eliminating member 106 or the bubble removing member remains below the blood surface (on or below the blood surface) in the blood storing portion 110 even if the blood surface therein becomes the same height as the lower ends of the blood reserving portions 130a, 130b of the blood reserving members 103a, 103b.

In a further embodiment, the blood delivery mechanism-equipped blood reservoir tank 100 is equipped with a blood delivery mechanism that includes a blood storing portion 110, and blood reserving members 103a, 103b including blood reserving portions 130a, 130b that communicate with the blood storing portion 110. The blood reserving portions 130a, 130b reserve amounts of blood proportional to the amount of blood stored in the blood storing portion 110 when the amount of blood stored in the blood storing portion 110 becomes equal to or less than a predetermined value. The blood reserving portions 130a, 130b allow blood to flow out of interiors thereof when pressed from outside. The blood delivery mechanism further includes blood delivering drive units 104a, 104b that operate when blood is to be sent out of the interiors of the blood reserving members 103a, 103b. Each blood reserving member 103a, 103b includes a blood reserving member body portion 141a, 141b that is provided with an inwardly concavity surface portion 154a, 154b formed from a hard material into the shape of a bowl, and a flexible diaphragm 142a, 142b whose peripheral end portion is retained to a peripheral end portion of the concavity surface portion 154a, 154b of the blood reserving member body portion 141a, 141b in such a manner that the peripheral end portion of the flexible diaphragm 142a, 142b is inclined toward a central portion of the concavity surface portion 154a, 154b. The blood delivering drive unit 104a, 104b includes a blood reserving member-pressurizing portion 143a, 143b formed from an expandable sheet material, and a blood delivering fluid flow port 144a, 144b for expanding and contracting the blood reserving member-pressurizing portion 143a, 143b. The flexible diaphragm 142a, 142b is pressed against the concavity surface portion 154a, 154b of the blood reserving member body portion 141a, 141b, without forming any substantial bent point, when the blood reserving member-pressurizing portion 143a, 143b is expanded.

In a still further embodiment, the blood delivery mechanism-equipped blood reservoir tank 100 is equipped with a blood delivery mechanism that includes a blood storing portion 110, and blood reserving members 103a, 103b including blood reserving portions 130a, 130b that communicate with the blood storing portion 110. The blood reserving portions 130a, 130b reserve amounts of blood proportional to the amount of blood stored in the blood storing portion 110 when the amount of blood stored in the blood storing portion 110 becomes equal to or less than a predetermined value. The blood reserving portions 130a, 130b allow blood to flow out of interiors thereof when pressed from outside. The blood delivery mechanism further includes blood delivering drive units 104a, 104b that operate when blood is to be sent out of the interiors of the blood reserving members 103a, 103b. Each blood reserving member 103a, 103b includes a blood reserving member body portion 141a, 141b formed from a hard material, and a flexible diaphragm 142a, 142b whose peripheral end portion is retained to the blood reserving member body portion 141a, 141b. The blood delivering drive unit 104a, 104b includes a blood reserving member-pressurizing portion 143a, 143b formed from an expandable sheet material, and a blood delivering fluid flow port 144a, 144b for expanding and contracting (stretching) the blood reserving member-pressurizing portion 143a, 143b. The blood delivery mechanism-equipped blood reservoir tank 100 further includes a communication passage that connects a space 152a, 152b formed between the diaphragm 142a, 142b and the blood reserving member-pressurizing portion 143a, 143b to the outside.

In addition to the two blood reserving members 103a, 103b, the blood delivery mechanism-equipped blood reservoir tank 100 includes a blood reservoir tank portion 102 and blood flow passage portions 135a, 135b that connect between the blood reservoir tank portion 102 and the blood reserving members 103a, 103b, respectively. A heat exchanger-equipped artificial lung 105 is connected to a bottom surface of the blood reservoir tank portion 102 as in a hanging manner. A blood inlet of the heat exchanger-equipped artificial lung 105 is connected to a blood discharge opening 107 of the blood delivery mechanism-equipped blood reservoir tank 100 by a tube 151. In Figs. 11 and 13, the tube 151 is removed. Provided outward of the diaphragms 142a, 142b are the blood delivering drive units 104a, 104b that press the diaphragms 142a, 142b directly or indirectly when operated.

The blood storing portion 110 is a space formed by a blood keeping portion 129 of the blood reservoir tank portion 102 and a blood keeping portion 128 of the blood delivery instrument 103. In the blood delivery mechanism-equipped blood reservoir tank 100, the upper ends of the diaphragms 142a, 142b are substantially at the same height as a joint portion 181 between the blood reservoir tank portion 102 and the blood delivery instrument 103, so that when the blood surface in the blood storing portion 110 lowers approximately below a lower end of the blood keeping portion 129 of the blood reservoir tank portion 102 (that is, when the amount of blood in the blood storing portion 110 becomes equal to or less than a predetermined value), the blood reserving members 103a, 103b reserve amounts of blood proportional to the amount of blood stored in the blood storing portion 110, and blood can be intermittently forced out of the delivery blood reserving members 103a, 103b by pressing the diaphragms 142 thereof from outside. Therefore, if the amount of blood in the blood storing portion 110 becomes small, blood is delivered in proportionally small amounts. Thus, if the amount of blood remaining becomes small, blood delivery is maintained in small amounts. Consequently, the need to interrupt blood delivery due to a reduction in the amount of blood remaining is eliminated, so that stagnation of blood in the extracorporeal blood circulation circuit can be avoided.

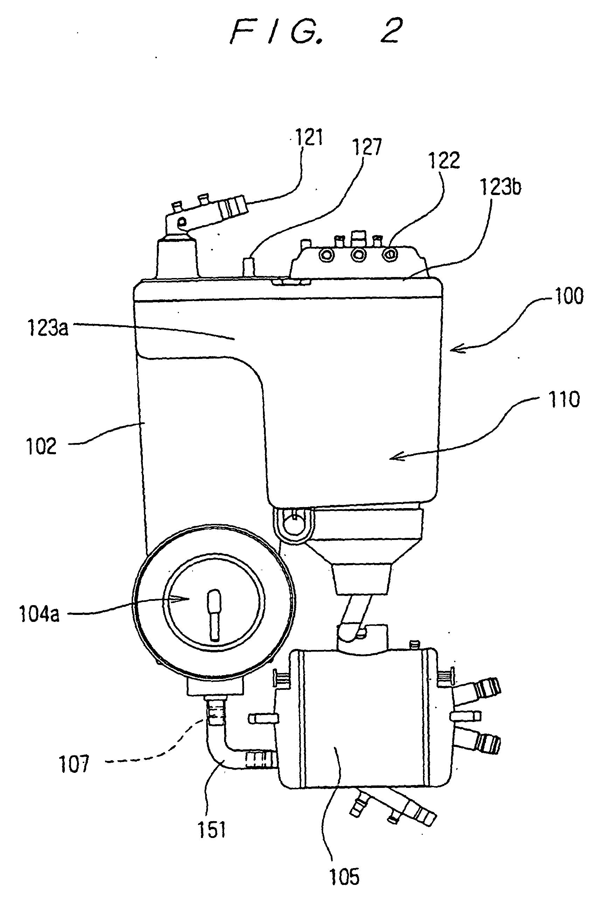

The blood reservoir tank portion 102 has a housing, as shown in Figs. 1-3, which is composed of a blood reservoir tank portion housing main body 123a and a lid body 123b that are formed from a hard resin. The lid body 123b is fitted to an upper end of the housing main body 123a so as to cover an upper opening of the housing body 123a. The lid body 123b has blood inlets 121, 122 and an air discharge opening 127 as shown in Figs. 1 and 2. The blood inlet 122 is connected to a cardiotomy line for conveying blood from an operation field. The blood inlet 121 is connected to a blood drainage line for conveying blood from a drainage cannula inserted into the heart ascending/descending veins of the patient.

The housing main body 123a and the lid body 123b may suitably be formed from, for example, polycarbonate, acrylic resin, polyethylene terephthalate, polyethylene, polypropylene, polystyrene, polyvinyl chloride, acryl-styrene copolymers, and acryl-butadiene-styrene copolymers, and the like. Particularly preferred among these are polycarbonate, acrylic resin, polystyrene, and polyvinyl chloride.

Disposed in the housing main body 123a are a blood filter (venous blood filter) 106 that is a combination of a bubble eliminating member and a bubble removing member for filtering blood incoming from the blood inlet port 121, and a cardiotomy blood filter (not shown) for filtering blood incoming from the blood inlet port 122. A lower end of the blood inlet port 121 extends into the blood storing portion 110, and is disposed inside the interior of the blood filter 106, so that the entire blood incoming from the blood inlet port 121 passes through the blood filter 106 and then flows into the blood storing portion 110. Therefore, a construction is made such that the dripping of blood incoming from the blood inlet port 121 directly onto the blood surface in the blood storing portion 110 is prevented. More specifically, the blood filter 106 is fixed to the inner surface of the lid body 123b and extends toward a lower portion of the blood storing portion 110 so that the blood filter 106 encloses the lower end portion of the blood inlet port 121 protruding into the blood storing portion 110.

The housing main body 123a has a downward projected portion 123c. The lower end portion of the blood filter 106 extends further downward of the projected portion 123c.

The interior of the blood filter 106 is filled with a bubble eliminating member 106a or a bubble removing member for eliminating air bubbles that may be contained in incoming blood. To form the bubble eliminating member 106a or the bubble removing member, various porous materials may suitably be used, including, but not limited to, foamed materials such as foamed polyurethane, foamed polyethylene, foamed polypropylene, foamed polystyrene and the like, mesh, woven fabrics, non-woven fabrics, porous ceramics, sintered materials of resin and the like. Materials having relatively small air passage resistance (pressure loss) are particularly preferred. If a material having less air passage resistance, for example, a foamed material such as foamed polyurethane or the like or other porous material, is used, the pore size is preferably approximately within the range of 20 µm to 10 mm and, more preferably, approximately within the range of 50 µm to 5 mm.

It is preferred that the bubble eliminating member 106a carries thereon a bubble eliminating agent that destroys bubbles when contacting them. A preferred bubble eliminating agent is silicone (silica-compounded type, oil type or the like). Examples of the method for causing to the bubble eliminating member 106a to support a bubble eliminating agent include, but are not limited to, a method wherein a bubble eliminating member is dipped in a liquid containing a. bubble eliminating agent, and a method wherein a liquid containing a bubble eliminating agent is applied or sprayed to a bubble eliminating member, and then dried (for example, at 30°C for 180 minutes).

The exterior of the bubble eliminating member 106a is covered with a filter member 106b. The filter member 106b removes foreign matter or air bubbles from blood. A preferred material for the filter member 106b is a porous material having a sufficiently high blood permeability. Examples of the porous material include mesh (net) materials, woven fabrics, non-woven fabrics and the like. Such materials may be used alone or in any desired combination (particularly, in the form of a laminate). It is preferred to use one or a combination of two or more of macromolecular materials that include polyesters such as PET, PBT or the like, polyolefins such as polyethylene, polypropylene, rayon, Tetron®, nylon (polyamide), and the like. It is also possible to use metallic materials such as aluminum, stainless steel or the like. Particularly preferred are polyesters, polypropylene, polyethylene and stainless steel. The opening size of a mesh used for the filter member 106b is preferably about 15-300 µm and, more preferably, about 20-200 µm. It is also preferred to subject the filter member 106b to a plasma treatment or a hydrophilic treatment such as coating with a hydrophilic macromolecular material or the like, in order to improve blood permeability.

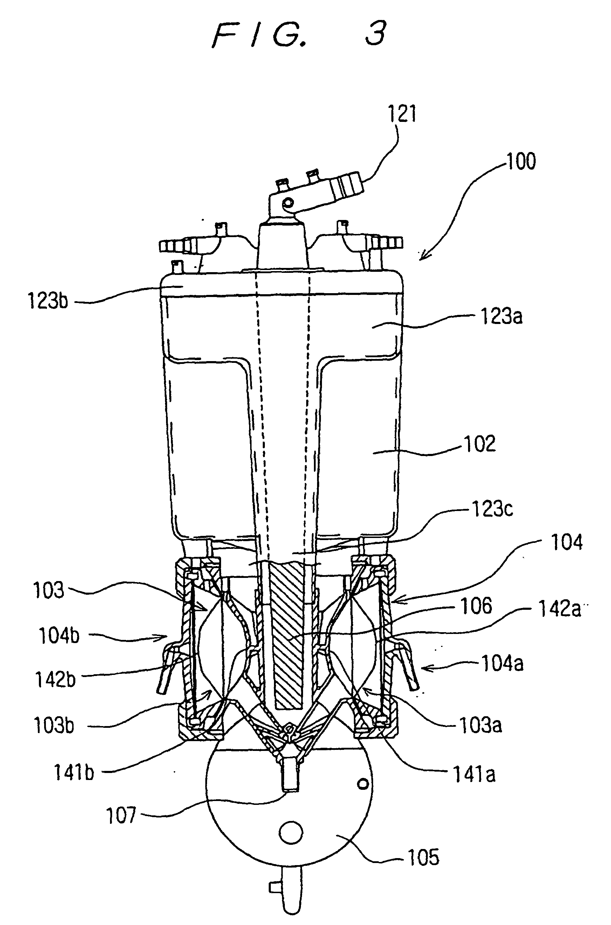

The interior of the blood reservoir tank portion 102 forms a portion of the blood storing portion 110 for temporarily reserving blood, as shown in Figs. 3 and 4. The capacity of the blood storing portion 110 is not particularly restricted. Normally, the capacity of the blood storing portion 110 is about 3,000 to 5,000 ml for adults and about 1,000 to 2,500 ml for children. The housing main body 123a is preferably substantially transparent or semi-transparent so that the amount and conditions of blood reserved therein may be readily observed. The downward projected portion 123c is a narrow portion with a reduced sectional area, whereby when the amount of blood reserved decreases, the amount of blood reserved or a change in the blood amount can be correctly and readily read. The blood reservoir tank portion 102 may be a soft type blood reservoir tank portion formed from a soft resin. Then, the blood reservoir tank portion 2 becomes a closed-type blood reservoir tank portion.

The blood delivery instrument 103 provided with the joint portion 181 to the blood reservoir tank portion 102 is firmly connected to a lower portion of the blood reservoir tank portion 102, as shown in Figs. 4-6. The blood delivery instrument 103 includes the blood keeping portion 128, which forms a portion of the blood storing portion 110, and the two blood reserving members 103a, 103b, and the blood flow passage portions 135a, 135b connecting the blood reserving members 103a, 103b to the blood keeping portion 128 (the blood storing portion 110), and the blood discharge opening 107.

A first blood flow passage portion 135a communicates only with a first delivery blood reserving member 103a, and the second blood flow passage portion 135b communicates only with the second delivery blood reserving member 103b. Disposed at boundary sites between the blood storing portion 110 and the blood flow passage portions 135a, 135b are first check valves 133a, 133b that allow flow of blood from the blood storing portion 110 toward the blood flow passage portions 135a, 135b (that is, toward the blood delivery instrument 103) and restricts (prevents) the reverse flow of blood.

The first check valves 133a, 133b function as passage control members for blocking the communication between the blood storing portion 110 and the delivery blood reserving members 103a, 103b during the operation of the blood delivering drive units 104 as described below. The blood delivery instrument 103 is provided with the blood discharge opening 107 for communication with the blood flow passage portions 135a, 135b. Provided near the blood discharge opening 107 are second check valves 134a, 134b that allow blood flow toward a side downstream from the blood flow passage portions 135a, 135b (that is, downstream from the blood delivery instrument 103) and restricts the reverse flow of blood. The second check valves 134a, 134b function as passage control members for blocking reverse blood flow from the downstream side toward the delivery blood reserving members 103a, 103b (that is, into the blood flow passage portions 135a, 135b) when the blood delivering drive unit 104 is not operated.

Each check valve 133, 134 has a disc-shaped movable valve body a portion of which is secured to the housing. Preferably, the movable valve body of each check valve has a slightly less specific gravity than blood, and a hardness of about 3 to 7 on Shore A scale. The valve bodies are preferably formed from, for example, styrene-based elastomer oil gel, silicone gel or the like, to a thickness of about 0.5 to 5 mm.

Each of the delivery blood reserving members 103a, 103b includes the delivery blood reserving member body part 141a, 141b formed from a hard material, the flexible diaphragm 142a, 142b supported or fixed at its peripheral end to the delivery blood reserving member body part 141, and the blood reserving portion 130a, 130b (Fig. 4) formed between the delivery blood storing member body part 141a, 141b and the flexible diaphragm 142a, 142b.

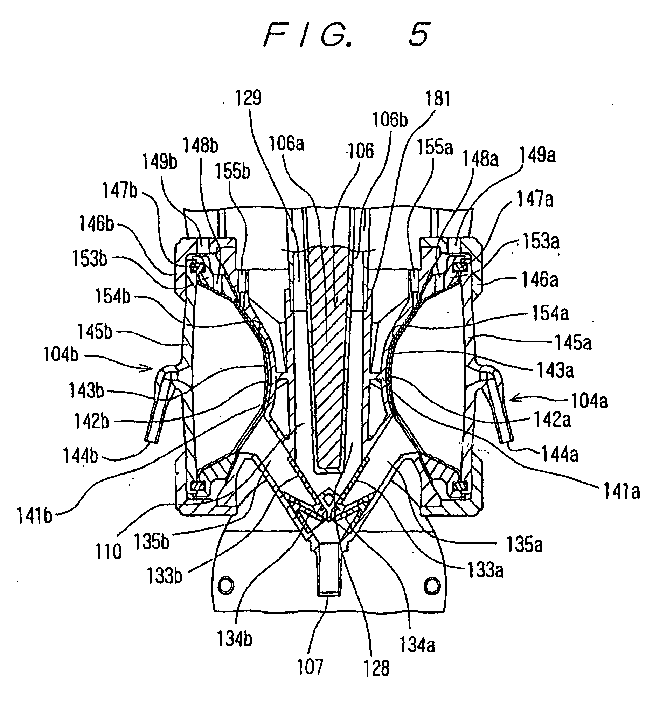

Fig. 4 is an enlarged sectional view of the blood delivery mechanism and a lower portion of the blood storing portion and their surroundings in the blood delivery mechanism-equipped blood reservoir tank shown in Fig. 1, wherein the flexible diaphragms 142a, 142b are not pressed (blood is not being delivered). Fig. 5 is an enlarged sectional view of the blood delivery mechanism and a lower portion of the blood storing portion and their surroundings in the blood delivery mechanism-equipped blood reservoir tank shown in Fig. 1, wherein the flexible diaphragms 142a, 142b are pressed (blood is being delivered). Fig. 6 is a further enlarged sectional view of a portion shown in Fig. 5.

Each blood reserving member 103a, 103b includes the flexible diaphragm 142a, 142b forming a portion of the blood reserving portion 130a, 130b. The flexible diaphragms 142a, 142b are formed from a flexible material. The blood delivery mechanism-equipped blood reservoir tank 100 is provided with the blood delivering drive units 104a, 104b that operate, at the time of blood delivery, to deform the flexible diaphragms 142a, 142b, that is, deformable portions, so as to force blood out of the blood reserving members 103a, 103b. The blood delivering drive units 104a, 104b will be described below.

Each blood reserving member body portion 141a, 141b is provided with the inwardly concavity surface portion 154a, 154b having the shape of a bowl. A peripheral end portion of each flexible diaphragm 142a, 142b is retained to a peripheral end portion of the concavity surface portion 154a, 154b of the blood reserving member body portion 141a, 141b in such a manner that the peripheral end portion of the flexible diaphragm 142a, 142b is inclined toward a central portion of the concavity surface portion 154a, 154b. The entire inner surface of each blood reserving member body portion 141a, 141b, including the peripheral end portion, is receded in the form of a bowl. This receded or concave surface of each blood reserving member body portion 141a, 141b closely contacts the flexible diaphragm 142a, 142b at the time blood delivery. Thus, each blood reserving portion 130a, 130b formed between the flexible diaphragm 142a, 142b and the concave surface of the blood reserving member body portion 141a, 141b is a space that is variable in capacity. Diaphragm retaining annular members 153a, 153b have a shape that conforms to the shape of an inclined peripheral end portion of the corresponding blood reserving member body portions 141a, 141b (a portion slightly inward from the peripheral edge). That is, each diaphragm retaining annular member 153a, 153b is formed so that it can be disposed within the peripheral end portion of the corresponding blood reserving member body portion 141a, 141b, and so that the diaphragm retaining annular member becomes substantially parallel to the inclined portion of the blood reserving member body portion 141a, 141b slightly inward from the peripheral end portion thereof. An outer peripheral surface of each diaphragm retaining annular member 153a, 153b protrudes in a taper form and faces the blood reserving member body portion 141a, 141b.

The peripheral end portion of each flexible diaphragm 142a, 142b is clamped between the peripheral end portion of the blood reserving member body portion 141a, 141b (a portion slightly inward from the peripheral end) and the outer peripheral portion of the diaphragm retaining annular member 133a, 153b. Corresponding to the shape of the clamping portion, the peripheral end portion of each flexible diaphragm 142a, 142b is inclined toward a central portion of the concavity surface portion 154a, 154b of the blood reserving member body portion 141a, 141b. Therefore, even when pressed against the concavity surface portion 154a, 154b of the blood reserving member body portion 141a, 141b, each flexible diaphragm 142a, 142b does not form a substantial bent point, that is, does not cause stress concentration at any point therein. Therefore, the incidence of damages to or breakage of the flexible diaphragms 142a, 142b is very low despite being repeatedly pressed against the concavity surface portions 154a, 154b of the blood reserving member body portions 141a, 141b.

The inner peripheral surface of each diaphragm retaining annular member 153a, 153b forms an inclined annular surface that is inclined toward a central portion of the concavity surface portion 154a, 154b of the blood reserving member body portion 141a, 141b and that is substantially flush with the concavity surface portion 154a, 154b, when the diaphragm retaining annular member 153a, 153b is set to clamp the flexible diaphragm 142a, 142b. Each blood reserving member-pressurizing portion 143a, 143b, formed of a sheet material for pressing the flexible diaphragm 142a, 142b as described below, is restricted at its peripheral end portion by the inclined annular surface of the diaphragm retaining annular member 153a, 153b, when expanded. Therefore, at the time of depression of the diaphragm as shown in Figs. 5 and 6, the blood reserving member-pressurizing portions 143a, 143b do not cause stress in the flexible diaphragms 142a, 142b, even at an annular contact end portion of each flexible diaphragm 142a, 142b (a portion adjacent to the distal peripheral end of the diaphragm retaining annular member 153a, 153b) at which stress is most likely to occur.

When the diaphragms are not depressed as shown in Fig. 4, each flexible diaphragm 142a, 142b bends at a clamped portion boundary therein (a portion adjacent to the distal peripheral end of the diaphragm retaining annular member 153a, 153b). However, in this state of the diaphragms, the flexible diaphragms 142a, 142b merely receive a pressure caused by a difference in height between the blood surfaces in the blood storing portion 110 and the blood reserving portions 130a, 130b, so that the flexible diaphragms 142a, 142b will not be damaged or broken.

Each blood reserving member 103a, 103b has an air vent port 155a, 155b that communicates with an upper portion of the blood reserving portion 130a, 130b. More specifically, each air vent port 155a, 155b is formed in an upper portion of the blood reserving member body portion 141a, 141b that is near the clamped portion boundary in the flexible diaphragm 142a, 142b. A tube provided with an open/close device, such as a three-way cock, is connected to each air vent port 155a, 155b. Using the tube, air removal is performed at the time of priming. It is also possible to connect an end of a tube with an open/close device, such as a three-way cock, connected to an intermediate portion thereof, to the air vent port 155a, 155b and the other end of the tube to an upper portion of the blood storing portion (a portion that normally remains above the blood surface). In this case, it becomes necessary to provide an upper side surface of the housing main body 123a or the lid body 123b with a connecting port. By providing such an air vent port communicating with an upper end of the blood reserving portion, it becomes very easy to remove air from the blood reserving member at the time of priming.

The maximum amount of blood that can be reserved by the delivery blood reserving members 103a, 103b (the maximum amount that can be contained therein, that is, the capacity thereof) varies depending on the capacity of the blood reservoir tank portion 102 used. However, the capacity of the delivery blood reserving members 103a, 103b is preferably about 20-500 mL and, particularly, about 50-500 mL. More preferably, the capacity thereof is about 50-300 mL and, particularly, about 80-300 mL.

The flexible diaphragms 142a, 142b have a shape corresponding to the shape of the inner surfaces of the concavity surface portions 154a, 154b of the blood reserving member body portions 141a, 141b. It is preferred that the pressure needed to deform each diaphragm 142a, 142b be equal to or less than 100 mmH₂O, whereby the flexible diaphragms 142a, 142b become more sensitive in responding to changes in the amount of blood stored in the blood storing portion 110, so that reservation of amounts of blood proportional to the amount of blood stored in the blood storing portion 110 is more reliably ensured. The pressure needed to deform each flexible diaphragm 142a, 142b is more preferably equal to or less than 50 mmH₂O.

The diaphragms 142a, 142b may be suitably formed from, for example, polyurethane, silicon-based polymers, polyvinyl chloride, and the like. Particularly preferred materials are polyurethane and silicon-based polymers, which are presently widely used for members that contact blood.

It is preferred that the diaphragms 142a, 142b be sufficiently soft. As an index of softness, compliance may be employed. The diaphragms 142a, 142b of the blood reserving members 103a, 103b have a compliance of, preferably, 2 ml/sec•mmHg or higher and, more preferably, within the range of 5-30 ml/sec•mmHg, when the blood surface in the blood storing portion 110 of the blood reservoir tank 100 is lower than the uppermost portion of the inner space of each of the delivery blood reserving members 103a, 103b, that is, when the pressure sensitivity (liquid surface sensitivity) of the delivery blood reserving members 103a, 103b is effective. It is further preferred that the diaphragms 142a, 142b of the delivery blood reserving members 103a, 103b have a compliance that is lower than the aforementioned value, when the blood surface in the blood storing portion 110 of the blood reservoir tank 100 is higher than the uppermost portion of the inner space of each of the delivery blood reserving members 103a, 103b. The resistance against blood inflow into the delivery blood reserving members 103a, 103b can be expressed by rate of blood inflow to the delivery blood reserving members 103a, 103b. The rate of blood inflow to the delivery blood reserving members 103a, 103b is preferably 20-600 ml/ sec.

It is preferred to provide each flexible diaphragm 142a, 142b with a reinforcement (not shown) which covers the outside of the diaphragm and whose peripheral end portion is fixed to the blood reserving member body portion 141a, 141b. Preferably, the diaphragm and the reinforcement combined have the following properties: the pressure needed to deform the diaphragm is equal to or less than 980Pa (100 mmH₂O); the rupture strength is equal to or greater than 5 kg/cm²; and the diaphragm itself produces substantially no self-restoring force against deformation. It is preferred that the diaphragm and the reinforcement of each delivery blood reserving member 103a, 103b be not adhered to each other at all or except at the peripheral end portions thereof. The reinforcements may be provided in the form of a woven fabric, a non-woven fabric, a knitted fabric, a sheet material or the like. In view of strength, the form of a woven or knitted fabric is preferred. As for examples of the material of the reinforcements, polyethylene terephthalate, nylon 6, nylon 66, regenerated cellulose, polypropylene, fiber-reinforced plastics (FRP, with aramid fiber or the like), stainless steel, aluminum or the like may be suitably used. Particularly preferred are polyethylene terephthalate, nylon 6, nylon 66, regenerated cellulose and polypropylene.

Each delivery blood reserving member 103a, 103b has, in addition to the blood reserving portion 130a, 130b provided therein, a blood flow opening 131a, 131b which is positioned at a lower portion of the delivery blood reserving member 103a, 103b (at a lower end thereof) and which communicates with the blood reserving portion 130a, 130b. Each blood reserving member 103a, 103b communicates with the blood flow passage portion 135a, 135b through the blood flow openings 131a, 131b. The delivery blood reserving members 103a, 103b are positioned above the first check valves 133a, 133b defining boundary sites between the blood storing portion 110 and the blood flow passage portions 135a, 135b. The delivery blood reserving members 103a, 103b extend substantially vertically upward. If the blood surface in the blood storing portion 110 is below the uppermost end of the blood reserving portion 130a, 130b (inner space) of each delivery blood reserving member 103a, 103b, amounts of blood proportional to the blood surface in the blood storing portion 110 flow into the delivery blood reserving members 103a, 103b. If the blood surface in the blood storing portion 110 is above the uppermost end of the blood reserving portion 130a, 130b (inner space) of each delivery blood reserving member 103a, 103b, the maximum amount of blood flows into the delivery blood reserving members 103a, 103b (as shown in Fig. 4) since the maximum capacity of the delivery blood reserving members 103a, 103b is fixed.

More specifically, the delivery blood reserving members 103a, 103b receive pressure proportional to the amount of blood stored in the blood storing portion 110, thus forming pressure-sensitive containers. If the amount of blood (liquid surface) in the blood storing portion 110 is equal to or greater than a predetermined amount (in this embodiment, the blood surface in the blood storing portion 110 is above the uppermost end of the inner space of each delivery blood reserving member 103a, 103b), the amount of blood reserved in the delivery blood reserving members 103a, 103b is determined by the maximum capacity thereof, not by the pressure corresponding to the amount of blood stored in the blood storing portion 110. That is, the delivery blood reserving members 103a, 103b reserve the maximum amount of blood. If the amount of blood stored in the blood storing portion 110 is equal to or less than the predetermined amount (in this embodiment, the blood surface in the blood storing portion 110 is below the uppermost end of the inner space of each delivery blood reserving member 103a, 103b), the pressure sensitivity of the delivery blood reserving members 103a, 103b becomes effective so that the delivery blood reserving members 103a, 103b reserve amounts of blood proportional to the amount of blood stored in the blood storing portion 110 (that is, the height of the blood surface in the blood storing portion 110). In short, the delivery blood reserving members 103a, 103b function to automatically reserve amounts of blood proportional to the amount of blood stored in the blood storing portion 110 when the amount of blood in the blood storing portion 110 is equal to or less than the predetermined value.

The delivery blood reserving members 103a, 103b do not spontaneously draw in blood, that is, do not substantially have self-shape-restoring characteristic. If the delivery blood reserving members 103a, 103b were formed so that the members have a preset shape and self-shape-restoring characteristic, the delivery blood reserving members 103a, 103b would restore their preset shape when the load from the drive members for forcing blood out of the members 103a, 103b is removed after blood is thereby forced out. Then the self-restoring force of the delivery blood reserving members 103a, 103b would cause a suction force, whereby blood would be drawn back into the delivery blood reserving members 103a, 103b from the side of the blood reservoir tank portion 102. In short, the delivery blood reserving members 103a, 103b would retain a certain minimum level of blood, below which the pressure sensitivity could not be effective.

In the blood delivery mechanism-equipped blood reservoir tank according to this embodiment, the blood filter 106 extends through the blood keeping portion 129 of the blood reservoir tank portion 102, to the blood keeping portion 128 of the blood delivery instrument 103. The lower end of the blood filter 106 is positioned below the lower ends of the blood reserving portions 130a, 130b. That is, the lower end of the blood filter 106 is positioned so as to remain below the blood surface in the blood storing portion 110 even if the blood surface lowers to the same height as the lower ends of the blood reserving portions 130a, 130b of the blood reserving members 103a, 103b.

Therefore, even when the amount of blood stored in the blood storing portion 110 decreases to or below a predetermined value so that the blood reserving members 103a, 103b function to reserve amounts of blood proportional to the amount of blood in the blood storing portion 110, there occurs substantially no bubbling due to flood inflow because the lower end of the blood filter 106 is positioned below the blood surface in the blood storing portion 110. For the same reason, substantially no or very little bubbling due to blood inflow occurs when the amount of blood in the blood storing portion 110 increases back to or above the predetermined value.

The capacity of each blood flow passage portion 135a, 135b (that is, a blood passage partially defined by the blood inlet of the blood reserving portion 130a, 130b and the first and second check valves 133a, 134a or 133b, 134b) is preferably equal to or less than 150 mL. Each blood flow passage portion 135a, 135b represents a blood passage partially defined by the first and second check valves 133a, 134a or 133b, 134b and the blood inlet of the blood reserving portion 130a, 130b of the blood reserving member 103a, 103b (the inner surface of the diaphragm 142a, 142b) when the flexible diaphragm 142a, 142b are closed or depressed.

The minimum sectional area of each blood flow passage portion 135a, 135b is equal to or greater than 1.0 cm². With such a sectional area of each blood flow passage portion 135a, 135b, the pressure sensitivity of the blood reserving members 103a, 103b will not be impeded, but can be made sufficiently high so that the blood reserving members 103a, 103b will reserve amounts of blood proportional to the amount of blood in the blood storing portion 110 (that is, the blood surface in the blood reservoir tank portion 102) when the amount of blood stored is equal to or less than a predetermined value. It is preferred that the pressure loss of each blood flow passage portion 135a, 135b (the pressure loss of a blood passage) be as small as possible. In this invention, the pressure loss is preferably 1270Pa (130 mmH₂O) or less and, particularly, 1080Pa (110 mmH₂O) and, more preferably, 980Pa (100 mmH₂O). With such a pressure loss, a sufficiently high pressure sensitivity and good operation can be achieved. The minimum sectional area of each blood flow passage portion 135a, 135b is more preferably 1.5 cm² or greater, whereby a favorable pressure sensitivity of the blood reserving members can be ensured.

It is also preferred that the capacity of each blood flow passage portion 135a, 135b be smaller than the maximum amount of blood that can be reserved in the blood reserving members 103a, 103b (the maximum amount that can be contained therein), whereby the entire volume of blood in the blood flow passage portion 135a, 135b can be forced out thereof by blood discharged from the blood reserving member 103a, 103b, thereby minimizing blood stagnation in the blood flow passage portion 135a, 135b.

The blood reserving member body portions 141a, 141b may suitably be formed from, for example, polycarbonate, acrylic resin, polyethylene terephthalate, polyethylene, polypropylene, polystyrene, polyvinyl chloride, acryl-styrene copolymers, and acryl-butadiene-styrene copolymers, and the like. Particularly preferred materials are polycarbonate, acrylic resin, polystyrene, and polyvinyl chloride. In this embodiment, the blood reserving member body portion 141a and the blood flow passage portion 135a are unitarily formed as a single member. Similarly, the blood reserving member body portion 141b and the blood flow passage portion 135b are unitarily formed. However, it is also possible to separately provide a blood reserving member body part and a blood flow passage part and then connect them.

The blood delivering drive units 104a, 104b are disposed outside the blood delivery instrument 103 in such a manner as to cover the blood reserving members 103a, 103b, respectively. The blood delivering drive units 104a, 104b and the blood delivery instrument 103 form a blood delivery mechanism.

Each blood delivering drive unit 104a, 104b is provided with the blood reserving member-pressurizing portion 143a, 143b formed of an expandable sheet material for operating to deliver blood out of the corresponding blood reserving member 103a, 103b. Each blood delivering drive unit 104a, 104b further includes a header 145a, 145b provided with the blood delivering fluid flow port 144a, 144b extending diagonally downwardly outward from a central portion of the header 145a, 145b, and a securing ring 146a, 146b for securing the header 145a, 145b to the blood reserving member body portion 141a, 141b.

Each blood reserving member-pressurizing portion 143a, 143b has an expanded or thickened peripheral end portion 147a, 147b. The thickened peripheral end portion 147a, 147b and an annular portion slightly inwardly of the thickened peripheral end portion 147a, 147b are clamped between the peripheral end portion of the header 145a, 145b and the peripheral end portion of the diaphragm retaining annular member 153a, 153b, and thereby substantially air-tightly retained. Each diaphragm retaining annular member 153a, 153b retains both the diaphragm 142a, 142b and the blood reserving member-pressurizing portion 143a, 143b, and controls excessive expansion of the diaphragm 142a, 142b. Since the blood reserving member-pressurizing portion 143a, 143b of each blood delivering drive unit 104a, 104b is an expandable or stretchable sheet member for expansion and contraction achieved by a fluid, the incidence of damaging or breaking the diaphragm by pressurization thereon is minimum. Furthermore, a relatively inward peripheral end portion of each blood reserving member-pressurizing portion 143a, 143b is restricted by the inclined annular surface of the diaphragm retaining annular member 153a, 153b when the blood reserving member-pressurizing portion 143a, 143b is expanded.

The inclined annular surface of each diaphragm retaining annular member 153a, 153b is inclined toward a central portion of the concavity surface portion 154a, 154b of the blood reserving member body portion 141a, 141b and that is substantially flush with the concavity surface portion 154a, 154b. Therefore, the blood reserving member-pressurizing portions 143a, 143b do not cause stress in the flexible diaphragms 142a, 142b, even at an annular contact end portion of each flexible diaphragm 142a, 142b (a portion adjacent to the distal peripheral end of the diaphragm retaining annular member 153a, 153b) at which stress is most likely to occur.

For use, a blood delivering fluid supply machine (not shown) is connected to the blood delivering fluid flow ports 144a, 144b. By supplying and discharging a fluid (liquid or gas) by a compressor provided in the blood delivering fluid supply machine, the expanding and contracting driving of the blood reserving member-pressurizing portions 143a, 143b is repeated. The blood reserving member-pressurizing portion 143a, 143b of each blood delivering drive unit 104a, 104b does not contact the diaphragm 142a, 142b of the blood reserving member 103a, 103b when not expanded (contracted) as shown in Fig. 4. When expanded, the blood reserving member-pressurizing portion 143a, 143b of each blood delivering drive unit 104a, 104b pressurizes the diaphragm 142a, 142b of the blood reserving member 103a, 103b in cooperation with the blood reserving member body portion 141a, 141b, thereby discharging blood out of the interior of the blood reserving member 103a, 103b (the interior of the blood reserving portion 130a, 130b).

The spaces 152a, 152b between the blood reserving members 103a, 103b (the diaphragms 142a, 142b) and the blood reserving member-pressurizing portions 143a, 143b (that is, spaces between the blood reserving members 103a, 103b and the blood delivering drive units 104a, 104b) are in communication with the outside. More specifically, each space 152a, 152b formed between the blood reserving member 103a, 103b and the blood reserving member-pressurizing portion 143a, 143b is in communication with the outside through a passage partially formed by an opening 148a, 148b that is formed in the diaphragm retaining annular member 153a, 153b (a component member of the blood delivering drive unit 104a, 104b) and an opening 149a, 149b that is formed in the securing ring 146a, 146b. Thus, in this embodiment, each blood delivering drive unit 104a, 104b has a passage for connecting the space between the blood reserving member 103a, 103b and the blood delivering drive unit 104a, 104b to the outside.

The shape and the manner of communication of the spaces 152a, 152b between the blood reserving members 103a, 103b and the blood reserving member-pressurizing portions 143a, 143b are not restricted by what is described above, as long as the spaces 152a, 152b communicate with the outside and do not form closed spaces. For example, if the securing rings 146a, 146b are not air-tightly fixed to the blood reserving member body portions 141a, 141b, communication passages for the spaces 152a, 152b may be formed by merely forming an opening only in each diaphragm retaining annular member 153a, 153b for communication with the outside. It is also possible to form a plurality of openings in a diaphragm retaining annular member 153a, 153b. In this case, the spaces 152a, 152b do not form tightly closed spaces, the spaces serve as decompression spaces when the blood reserving member-pressurizing portions 143a, 143b contract from an expanded state, thereby preventing the blood reserving members 103a, 103b from drawing in blood.

Preferred examples of the elastic material used to form the blood reserving member-pressurizing portions 143a, 143b include synthetic rubbers such as urethane rubber, silicone rubber, butadiene rubber and the like, natural rubbers such as latex rubber and the like, and elastomers such as olefin-based elastomer, amide-based elastomer, styrene-based elastomer (for example, styrene-butadiene-styrene copolymers, styrene-isoprene-styrene copolymers, styrene-ethylenebutylene-styrene copolymers), polyurethane elastomer and the like.

The blood delivering drive unit may adjust the amount of blood forced out of the blood reserving member by adjusting the amount or pressure of the fluid fed into the drive unit. The amount of blood delivered may be easily changed by adjusting the amount or pressure of the fluid fed into the blood delivering drive unit while fixing the number of times of driving the blood delivering drive unit in every predetermined length of time.

The blood delivery mechanism-equipped blood reservoir tank 100 of this embodiment is equipped with the two sets of the delivery blood storing member 103 and the blood delivering drive unit 104, as described above. Further, the two separate blood flow passage portions 135 (135a, 135b) are provided without communication therebetween, as shown in Figs. 4 and 5. By providing two or more sets thereof in this manner, the capacity of each delivery blood reserving member 103a, 103b and each blood delivering drive unit 104a, 104b can be reduced, so that the blood inflow and discharge response improves. Furthermore, if two or more sets of the delivery blood storing member 103 and the blood delivering drive unit 104 are provided, good blood flow may be achieved by shifting the expanding strokes of the blood delivering drive units 104a, 104b in timing from each other. If the expanding timing of the blood delivering drive units 104a, 104b is thus shifted, flow of blood from one blood reserving member to the other in this embodiment never occurs since the individual blood reserving members are provided with the separate blood flow passage portions 135a, 135b. The construction of the invention is not limited by this embodiment. The number of sets of the delivery blood reserving member and the blood delivering drive unit may also be one, or three or more. In addition, although the blood delivering drive units 104a, 104b in this embodiment repeat expansion and contraction through fluid operation, the blood delivering drive unit is not restricted by this driving manner. For example, the blood delivering drive unit may mechanically drive a pressurizing plate to pressurize the blood reserving member.

If two sets of the delivery blood reserving member and the blood delivering drive unit are provided as in blood delivery mechanism-equipped blood reservoir tank 100 and, furthermore, the individual blood delivering drive units can be separately controlled by a fluid supply machine for blood delivery, it becomes possible to select the form of blood flow for delivery, more specifically, select a pulse flow or a constant flow, considering the conditions of a patient, the type of the artificial lung used or the like. It becomes also possible to change the form of delivery blood flow while in use. More specifically, a substantially constant blood flow can be achieved by shifting the phases of the blood flow out of the individual delivery blood reserving members 103a, 103b by substantially 180° from each other, that is, by shifting the phases of fluid flowing into and out of the blood delivering drive units 104a, 104b by substantially 180° from each other. A good pulse flow can be achieved by setting the phases of blood flow out of the individual blood reserving members 103a, 103b to substantially the same phase or within ±30°, that is, by setting the phases of fluid flowing into and out of the blood delivering drive units 104a, 104b to substantially the same phase or within ±30°. If three or more sets of the delivery blood storing member and the blood delivering drive unit are provided, the form of blood flow will become a substantially constant flow by shifting the phases of blood flow out of the individual delivery blood reserving members by an angle obtained by dividing 360° by the number of the sets provided.

In the blood delivery mechanism-equipped blood reservoir tank 100, the diaphragms 142a, 142b of the blood reserving members 103a, 103b are surrounded by the headers 145a, 145b and the like, and are not exposed to outside. Thus, the function of protective covers for the diaphragms are provided.

The blood delivery mechanism-equipped blood reservoir tank of the present invention may be provided as a blood delivery mechanism-equipped blood reservoir tank 200 as shown in Figs. 10 and 11. Fig. 10 is a partially cut-away side view of a blood delivery mechanism-equipped blood reservoir tank according a further embodiment of the present invention. Fig. 10 is a sectional view of a lower portion of the blood delivery mechanism-equipped blood reservoir tank shown in Fig. 10, taken on a horizontal plane, wherein a case member is removed.

The blood delivery mechanism-equipped blood reservoir tank 200 of this embodiment differs from the blood delivery mechanism-equipped blood reservoir tank 100 of Fig. 1 to 6 in the mechanism of blood reserving members and blood reserving member-pressurizing portions.

Similar to the blood delivery mechanism-equipped blood reservoir tank 100 of Fig. 1 to 6, the blood delivery mechanism-equipped blood reservoir tank 200 of this embodiment of the invention includes a blood storing portion 210, and a blood reserving member including a blood reserving portion that communicates with the blood storing portion 210. The blood reserving portion reserves amounts of blood proportional to the amount of blood stored in the blood storing portion when the amount of blood stored in the blood storing portion is equal to or less than a predetermined value. The blood reserving portion allows blood to flow out of the interior thereof when pressed from outside. The blood reserving member is a soft tubular body 203. A pump 204 is provided as a blood reserving member-pressurizing member for pressurizing the soft tubular body 203 to force blood out of the interior of the soft tubular body 203. The blood delivery mechanism-equipped blood reservoir tank 200 further includes a case member 211 that substantially air-tightly separates, from the outside, the soft tubular body 203 and a pumping portion for pressurizing the soft tubular body 203 to deliver blood therefrom, and a communication passage (formed by a communication member 185) connecting the interior of the case member 211 and an upper portion of the blood storing portion in communication.

In the blood delivery mechanism-equipped blood reservoir tank 200, the blood reserving member is formed by the soft tubular body 203 extending horizontally. The soft tubular body 203 has substantially no self-restoring characteristic, as in the blood reserving members described above. Therefore, if the amount of blood stored in the blood storing portion 210 decreases so that the blood surface in the blood storing portion 210 becomes lower than an upper end of the soft tubular body 203, the soft tubular body 203 reserves amounts of blood corresponding to the height of blood surface, thus performing pressure-sensitive operation. The soft tubular body 203 is connected with a tube 207 that forms a blood discharge opening.

Employed as a blood reserving member-pressurizing member is the pump 204, which pressurizes the soft tubular body 203 to force blood out of the interior thereof. The pump 204 in this embodiment is a roller pump. The pump may also be a peristaltic pump. The pump 204 delivers blood from the soft tubular body 203 in a squeezing manner such that a squeezed or compressed portion achieves a closed state in the soft tubular body 203. Therefore, the two check valves provided in the blood delivery mechanism-equipped blood reservoir tank 100 of the foregoing embodiment are not necessary in this embodiment. The blood delivery mechanism-equipped blood reservoir tank 200 further includes a soft tubular body-supporting portion 205. The soft tubular body 203 is squeezed between the soft tubular body-supporting portion 205 and a roller 204a of the roller pump 204.

As in the blood delivery mechanism-equipped blood reservoir tank 100, a bubble eliminating member 106 in the blood delivery mechanism-equipped blood reservoir tank 200 extends to a proximity of a bottom of the blood reservoir tank portion 102, and the lower end of thereof is positioned below a lower end of the soft tubular body 203, that is, a blood reserving member. In other words, the blood filter 106 is provided so that the lower end thereof remains below the blood surface (on or below the blood surface) in the blood storing portion 210 even if the blood surface therein becomes the same height as the lower ends of the soft tubular body 203.

Therefore, even when the amount of blood stored in the blood storing portion 210 decreases to or below a predetermined value so that the soft tubular body 203 functions to reserve amounts of blood proportional to the amount of blood in the blood storing portion 210, there occurs substantially no bubbling due to flood inflow because the lower end of the blood filter 106 is positioned below the blood surface in the blood storing portion 210. For the same reason, substantially no or very little bubbling due to blood inflow occurs when the amount of blood in the blood storing portion 210 increases back to or above the predetermined value.

The blood delivery mechanism-equipped blood reservoir tank 200 may also be provided with two or more sets of the blood reserving member and the blood reserving member-pressing member:

The blood delivery mechanism-equipped blood reservoir tank 200 is provided with the case member 211 that substantially air-tightly separates, from the outside, the soft tubular body 203 and a pumping portion for pressurizing the soft tubular body 203 to deliver blood therefrom, and the communication passage 185 connecting the interior of the case member 211 and an upper portion of the blood storing portion 210 in communication. More specifically, the case member 211 is mounted on the pump main body 204, and substantially air-tightly separates the soft tubular body-supporting portion 205 and the roller 204a and the like of the roller pump 204 from the outside. Therefore, the space enclosing at least the soft tubular body 203 is not in direct communication with the outside. As shown in Fig. 20, the blood delivery mechanism-equipped blood reservoir tank 200 has a suction device connecting port 182, and a communication member connecting port 212 forming a communication passage (communicating with the blood storing portion 201). The case member 211 has a communication member connecting port 191. The communication member connecting port 212 is provided with an air-permeable and blood-impermeable member (not shown). The blood-impermeable member may be a membrane filter, a sintered filter or the like. It is preferred that the pressure loss of the blood-impermeable member be small. The communication member connecting port 212 may be positioned at any site where the possibility of contact with blood is small. Although the communication member connecting port 212 is provided so as to communicate with an uppermost end of the interior of the blood reservoir tank in Fig. 10, this disclosed construction is not restrictive. For example, the communication member connecting port 212 may also be provided in an upper portion of a housing 123a of the blood reservoir tank. The case member 211 has an opening for air-tightly holding the tube 207. (extending therethrough). The opening of the case member 211 is provided with an O-ring 208 formed from an elastomer or rubber such as silicone rubber, urethane rubber or the like.

The communication member connecting ports 191 and 212 are connected by the communication member 185. The communication member 185 may be a hard or soft tube.

Lately, the conveyance of blood into the blood reservoir tank and the drainage of blood from a patient are often performed by a method using a suction device (negative pressure applying device) connected to the blood reservoir tank. When this device is operated, the pressure in the blood reservoir tank portion becomes negative, and the negative pressure affects the interior of a soft tubular body. That is, the soft tubular body becomes exposed to a negative pressure inside due to the operation of the suction device (negative pressure applying device), while receiving the atmospheric pressure on the outside. Therefore, the soft tubular body is pressed by a differential pressure between the inside and outside pressures, so that the liquid surface-sensitive (pressure-sensitive, pre-load-sensitive) function of the soft tubular body, that is, the delivery blood reserving member, may be impeded. However, in the aforementioned construction in which the space (the interior of the case member 211) enclosing the blood storing portion 201 and the soft tubular body 203 are connected by the communication member 185, when suction from the blood reservoir tank portion is performed so that the pressure in the blood reservoir tank portion becomes negative, the pressure in the case member 211 simultaneously becomes negative, thereby avoiding the impeding of the pre-load-sensitivity performance of the soft tubular body 203 corresponding to the amount of blood stored (the height of liquid surface). The communication member connecting port 191 may also be provided with an air-permeable and blood-impermeable member (not shown). The blood-impermeable member may be a membrane filter, a sintered filter or the like. It is preferred that the pressure loss of the blood-impermeable member be small.

A still further embodiment of the blood delivery mechanism-equipped blood reservoir tank of the invention will be described with reference to Figs. 7-9.

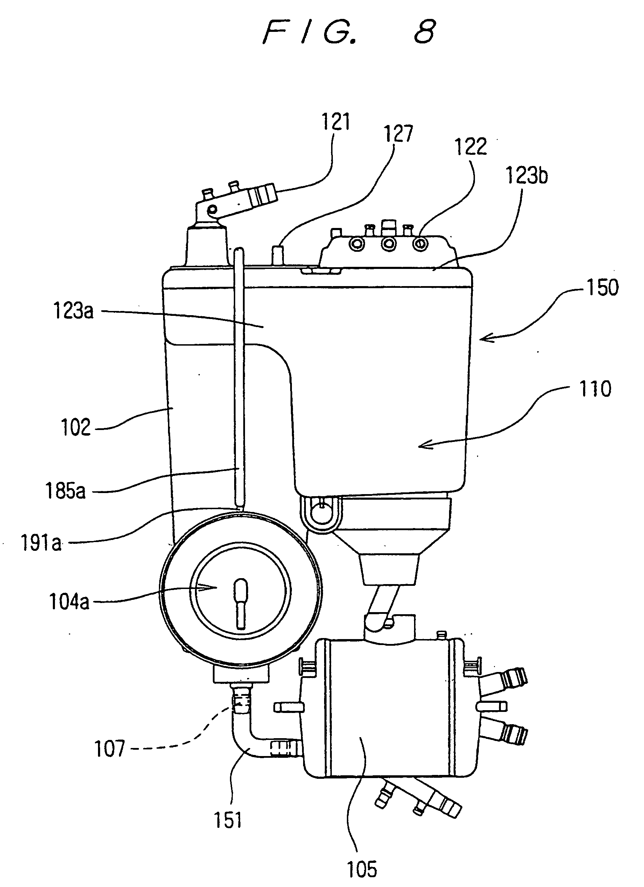

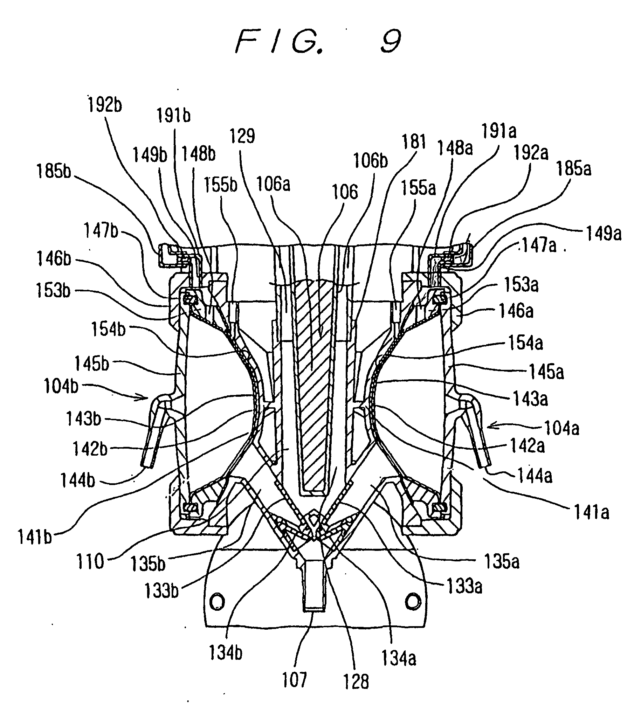

Fig. 7 is a front elevation of a blood delivery mechanism-equipped blood reservoir tank 150 according to an embodiment of the present invention, with an artificial lung connected. Fig. 8 is a side view of the blood delivery mechanism-equipped blood reservoir tank according to the embodiment, with the artificial lung connected. Fig. 9 is an enlarged sectional view of the blood delivery mechanism and a lower portion of a blood storing portion and their surroundings in the blood delivery mechanism-equipped blood reservoir tank shown in Fig. 7, at the time of blood delivery.

The blood delivery mechanism-equipped blood reservoir tank 150 of this embodiment is substantially the same in a basic construction as the blood delivery mechanism-equipped blood reservoir tank 100 described above. Like portions are represented by like reference characters in the drawings, and will not be described again. The blood delivery mechanism-equipped blood reservoir tank 150 differs from the blood delivery mechanism-equipped blood reservoir tank 100 in that the blood delivery mechanism-equipped blood reservoir tank 150 includes a communication passage for communication between a space formed between the diaphragm and the blood reserving member-pressurizing portion and an upper portion of the blood storing portion. Therefore, the space between the diaphragm and the blood reserving member-pressurizing portion is not in direct communication with the outside in this embodiment. The communication passage will be described in detail below.

In the blood delivery mechanism-equipped blood reservoir tank 150, a lid body 123b has, as shown in Figs. 7 and 8, a suction device connecting port 182, and communication ports 182a, 182b for connecting to passages that are formed in blood delivering drive units 104a, 104b so as to connect spaces 152a, 152b between the diaphragms and the blood reserving member-pressurizing portions, to the outside. The communication ports 182a, 182b are provided with air-permeable and blood-impermeable members (not shown). The blood-impermeable members may be membrane filters, sintered filters or the like. It is preferred that the pressure loss of the blood-impermeable members be small. The communication ports 182a, 182b may be positioned at any sites where the possibility of contact with blood is small. Although the communication ports 182a, 182b are provided so as to communicate with uppermost end portions of the interior of the blood reservoir tank in Figs 17 and 18, this disclosed construction is not restrictive. For example, the communication ports 182a, 182b may also be provided in upper portions of a housing 123a of the blood reservoir tank. Communication members 185a, 185b are connected to the communication ports 182a, 182b, respectively. The communication members 185a, 185b form communication passages connecting passages (openings 149a, 149b) of the blood delivering drive units 104a, 104b to an upper portion of the blood storing portion. The communication members 185a, 185b may be hard or soft tubes.

The portions between the delivery blood reserving members 103a, 103b and the corresponding blood delivering drive units 104a, 104b are air-tightly sealed, except for the passages (openings 148a, 148b and the openings 149a, 149b). Therefore, the spaces 152a, 152b between the delivery blood reserving members 103a, 103b and the blood delivering drive units 104a, 104b communicate with the outside only through those passages.

In this embodiment, communication ports 191a, 191b are connected to the openings 148a, 148b formed in diaphragm retaining annular members 153a, 153b, for communication with the blood storing portion in the blood reservoir tank portion. The communication ports 191a, 191b are in communication with the aforementioned communication ports 182a, 182b through the communication members 185a, 185b. Therefore, the blood storing portion is in communication with the spaces 152a, 152b formed between the delivery blood reserving members 103a, 103b (the diaphragms 142a, 142b) and the blood reserving member-pressurizing portions 143a, 143b.

Lately, the conveyance of blood into the blood reservoir tank and the drainage of blood from a patient are often performed by a method using a suction device (negative pressure applying device) connected to the blood reservoir tank. When this device is operated, the pressure in the blood reservoir tank portion becomes negative, and the negative pressure affects the interiors of delivery blood reserving members. That is, the delivery blood reserving members become exposed to a negative pressure inside due to the operation of the suction device (negative pressure applying device), while receiving the atmospheric pressure on the outside. Therefore, the delivery blood reserving members are pressed by a differential pressure between the inside and outside pressures, so that the liquid surface-sensitive (pressure-sensitive, pre-load-sensitive) function of the delivery blood reserving members may be impeded. However, in the aforementioned construction in which the blood storing portion 110 (blood keeping portion 129 in the blood reservoir tank portion 102) is in communication with the spaces 152a, 152b formed between the delivery blood reserving members 103a, 103b (the diaphragms 142a, 142b) and the blood reserving member-pressurizing portions 143a, 143b through the communication members 185a, 185b, when suction from the blood reservoir tank portion 102 is performed so that the pressure in the blood reservoir tank portion 102 becomes negative, the pressure in the spaces 152a, 152b between the delivery blood reserving members 103a, 103b (the diaphragms 142a, 142b) and the blood reserving member-pressurizing portions 143a, 143b simultaneously becomes negative. This construction thus avoids the impeding of the pre-load-sensitivity performance of the delivery blood reserving members 103a, 103b corresponding to the amount of blood stored in the blood storing portion 110 (the height of liquid surface therein). The positions of the communication ports 191a, 191b, that is, the positions of the passages, may be any positions as long as the ports or passages communicate with the spaces 152a, 152b. The communication ports 191a, 191b may also be provided with air-permeable and blood-impermeable members (not shown). The blood-impermeable members may be membrane filters, sintered filters or the like. It is preferred that the pressure loss of the blood-impermeable members be small.

Where the communication members 185a, 185b are provided, a closed space is formed by the spaces 152a, 152b and the blood storing portion 110. It is expected that the pressure in the closed space will slightly decrease when the blood reserving member-pressurizing portions 143a, 143b contract from the expanded state. However, the pressure in the blood storing portion 110 decreases as the pressure in the spaces 152a, 152b decreases, so that no pressure difference occurs across the diaphragms. Therefore, suction of blood into the delivery blood reserving members 103a, 103b by a reduced pressure will not occur.

In a blood delivery mechanism-equipped blood reservoir tank according to the invention, the lower end of the bubble eliminating member or the bubble removing member is positioned below the lower end of the blood reserving portion of the blood reserving member.

Furthermore, in a blood delivery mechanism-equipped blood reservoir tank according to the invention, the lower end of the bubble eliminating member or the bubble removing member is positioned so as to remain below the blood surface in the blood storing portion even when the blood surface therein lowers to the same height as the lower end of the blood reserving portion of the blood reserving member.

Therefore, even when the amount of blood stored in the blood storing portion decreases to or below a predetermined value so that the blood reserving member functions to reserve amounts of blood proportional to the amount of blood in the blood storing portion, there occurs substantially no bubbling due to flood inflow because the lower end of the bubble eliminating member of the bubble removing member is positioned below the blood surface in the blood storing portion. For the same reason, substantially no or very little bubbling due to blood inflow occurs when the amount of blood in the blood storing portion increases back to or above the predetermined value.

In a blood delivery mechanism-equipped blood reservoir tank according to the invention, the blood reserving member includes a body portion of the blood reserving member which body portion is provided with a concavity surface portion formed from a hard material, and a flexible diaphragm whose peripheral end portion is retained adjacent to a peripheral end portion of the concavity surface portion of the body portion of the blood reserving member so that the peripheral end portion of the diagram is inclined toward a central portion of the concavity surface portion. The blood delivering drive unit includes a blood reserving member-pressurizing portion formed from an expandable sheet material, and a blood delivering fluid flow port for expanding and contracting the blood reserving member-pressurizing portion. The flexible diaphragm is pressed against the concavity surface portion of the body portion of the blood reserving member, without forming any substantial bent point, when the blood reserving member-pressurizing portion is expanded.

Therefore, even when pressed against the concavity surface portion of the blood reserving member body portion, the flexible diaphragm does not form a substantial bent point, that is, does not cause stress concentration at any point therein. Therefore, the incidence of damages to or breakage of the flexible diaphragms is very low despite being repeatedly pressed against the concavity surface portion of the blood reserving member body portion. That is, the diaphragm can be continually used for an extended period.

It is preferred that the blood contact surfaces in all the delivery blood storing member-equipped blood reservoir tanks, the blood delivery mechanism-equipped blood reservoir tanks and the blood delivery instruments for an extracorporeal blood circulation circuit described above be non-thrombogenic surfaces. Particularly, the blood contact surfaces of the delivery blood storing members or the delivery blood reserving members, the blood passages, the first check valves and the second check valves are preferred to be non-thrombogenic surfaces.

A non-thrombogenic surface may be formed by subjecting a surface to the coating treatment with a non-thrombogenic material followed by fixation thereof. Examples of the non-thrombogenic material that may suitably be used are heparin, urokinase, HEMA-St-HEMA copolymers, poly-HEMA, and the like.

Preferably, the non-thrombogenic surface is formed by treating a substrate with ozone to form functional group-bearing oxides on the substrate surface and applying heparin to the surface so that an amino group of heparin forms a covalent bond with the functional group directly or through at least one coupling agent. This method allows heparin to be fixed on the blood contact surface without the use of a solvent, thereby minimizing changes of physical properties (e.g., flexibility, elasticity and strength) of the substrate presenting the blood contact surface.

Through ozone treatment, oxides are formed on the substrate surface, containing various functional groups, for example, high reactive functional groups such as aldehyde, ketone, and epoxy groups.

Functional groups of heparin can directly bond with these functional groups. However, such direct bonding method may have problems of steric hindrance and the like. A method wherein a spacer (coupling agent) is introduced to the functional groups prior to fixation of heparin is easy and useful in view of development of the heparin activity. Either a coupling agent or a combination of two or more coupling agents may be used. Compounds having at least two aldehyde or epoxy groups are preferred as coupling agents.

Where two or more coupling agents are used, it is preferred that a coupling agent (spacer coupling agent) of a compound having at least two functional groups, such as amino groups, be first bonded with the functional groups previously formed on the substrate to form amino acid or the like on the substrate, and then heparin be bonded with the substrate using a coupling agent (heparin-fixing coupling agent) of a compound having at least two aldehyde or epoxy groups. In bonding heparin, the coupling agent is preferably introduced into the reaction system simultaneously with or subsequent to introduction of heparin.

If an amino group is introduced using a spacer coupling agent, the amino group exhibits substantially the same reactivity as the amino group of heparin in the reaction system, so that subsequent fixation of heparin to the substrate by the heparin-fixing coupling agent will take place more effectively.

Where the functional group of a coupling agent to directly bond with heparin or the functional group introduced into the substrate is aldehyde group, it is preferable to use heparin in which some N-sulfate groups are desulfurized into primary amino groups.

It is preferred that the spacer coupling agent be one that forms a bond (covalent bond) with the functional group formed on the substrate by ozone treatment and have at least two primary amino groups. Examples of the spacer coupling agent having at least two amino groups include polyethylene imine (PEI), polyethylene glycol diamine, ethylene diamine, and tetramethylene diamine, and the like.

Aldehyde compounds and epoxy compounds are preferable as a coupling agent used for fixing heparin to the substrate. Preferred examples of the aldehyde compounds are glutaraldehyde, glyoxal, succindialdehyde, and the like. Preferred examples of the epoxy compounds are polyethylene glycol diglycidyl ether, 1,4-butanediol-diglycidyl ether, sorbitol diglycidyl ether, glycerol diglycidyl ether, and the like.

Illustrative examples may be: Denacol EX-421, 521, 611, 612, 614, and 614B where the epoxy compound is sorbitol diglycidyl ether; Denacol EX-313 where the diepoxy compound is glycerol diglycidyl ether; Denacol EX-810, 811, 851, 821, 830, 832, 841, and 861 where the diepoxy compound is polyethylene glycol diglycidyl ether, all commercially available from Nagase Chemicals K.K. Denacol EX-313, 421, 512, 521, 810, 811, 821, and 851 are preferred when the difference in epoxy reactivity is considered. In the above-mentioned heparin fixation, the coupling-off of heparin is minimized since the bond between polyethylene imine fixed to the substrate and glutaraldehyde and the bond between glutaraldehyde and heparin are both covalent bonds.

While the present invention has been described with reference to what are presently considered to be preferred embodiments thereof, it is to be understood that the invention is not limited to the disclosed embodiments or constructions. To the contrary, the invention is intended to cover various modifications as for as these are included within scope of the appended claims.

## Claims

1. A blood reservoir tank (100; 150) equipped with a blood delivery mechanism, the tank comprising:
a blood storing portion (110);
a blood reserving member (103a, 103b) including a blood reserving portion (130a, 130b) that communicates with the blood storing portion, the blood reserving portion reserving an amount of blood proportional to an amount of blood stored in the blood storing portion when the amount of blood stored in the blood storing portion (110) becomes equal to or less than a predetermined value, the blood reserving portion allowing blood to flow out of an interior thereof when pressed from outside; and
a blood delivering drive unit (104a, 104b) that operates when blood is to be sent out of the interior of the blood reserving portion,
the blood reserving member including a body portion (141a, 141b) of the blood reserving member which body portion is formed from a hard material, and a flexible diaphragm (142a, 142b) whose peripheral portion is retained to the body portion of the blood reserving member,
the blood delivering drive unit (104a, 104b) including a blood reserving member-pressurizing portion (143a, 143b) formed from an expandable sheet material, and a blood delivering fluid flow port (144a, 144b) for expanding and contracting the blood reserving member-pressurizing portion,
**characterized by**
a communication passage (185a, 185b) connecting a space (152a, 152b) formed between the diaphragm (142a, 142b) and the blood reserving member-pressurizing portion (143a, 143b) to an upper portion of the blood storing portion (110).

2. A blood delivery mechanism-equipped blood reservoir tank according to claim 1, wherein the blood reserving member (103a, 103b) comprises an air vent port that communicates with an upper end of the blood reserving portion (130a, 130b).

3. A blood delivery mechanism-equipped blood reservoir tank (200) comprising:
a blood storing portion (210);
a blood reserving member (203) including a blood reserving portion that communicates with the blood storing portion, the blood reserving portion reserving an amount of blood proportional to an amount of blood stored in the blood storing portion when the amount of blood stored in the blood storing portion (210) becomes equal to or less than a predetermined value, the blood reserving portion allowing blood to flow out of an interior thereof when pressed from outside; and
a blood reserving member-pressurizing portion (204) that operates when blood is to be sent out of the blood reserving member, **characterized by**
the blood reserving member being a soft material-made tubular body (203),
the blood reserving member-pressurizing portion being a pump (204) that compresses the soft material-made tubular body to force blood out of an interior of the tubular body,
a case member (211) separating the soft material-made tubular body (203) and a pump site that is a portion that compresses the soft material-made tubular body to force blood out of the interior of the tubular body, airtightly from the outside; and
a communication passage (185) connecting an interior of the case member (211) to an upper portion of the blood storing portion (210).

## Patentansprüche

1. Ein mit einem Blutabgabemechanismus ausgerüsteter Blutreservoirbehälter (100; 150),
wobei der Behälter aufweist:
einen Blutaufbewahrungsbereich (110);
ein Blutvorhalteteil (103a, 103b), das einen Blutvorhaltebereich (130a, 130b) enthält, der mit dem Blutaufbewahrungsbereich verbunden ist, wobei der Blutvorhaltebereich eine Menge an Blut vorhält, die proportional zu der Menge des in dem Blutaufbewahrungsbereich aufbewahrten Blutes ist, wenn die Menge des in dem Blutaufbewahrungsbereich (110) aufbewahrten Blutes gleich oder kleiner einem vorbestimmten Wert wird, wobei es der Blutvorhaltebereich erlaubt, dass Blut aus seinem Innenraum ausströmt, wenn von außen unter Druck gesetzt; und
eine Antriebseinheit (104a, 104b) zur Abgabe von Blut, die betrieben wird, wenn Blut aus dem Innenraum des Blutvorhaltebereichs ausgetrieben werden soll,
wobei das Blutvorhalteteil einen Rumpfteil (141a, 141b) des Blutvorhalteteils, wobei der Rumpfteil aus einem festen Material gemacht ist, und eine flexible Membran (142a, 142b) enthält, deren Randbereich an dem Rumpfteil des Blutvorhalteteils gehalten ist,
wobei die Antriebseinheit (104a, 104b) zur Abgabe von Blut einen aus einem dehnbaren flächigen Material gebildeten Bereich (143a, 143b) zum Unterdrucksetzen des Blutvorhalteteils und eine Durchflussöffnung (144a, 144b) für eine Blutabgabe-Flüssigkeit zum Expandieren und Kontrahieren des Bereichs zum Unterdrucksetzen des Blutvorhalteteils enthält, **gekennzeichnet durch**
einen Verbindungsweg (185a, 185b), der einen zwischen der Membran (142a, 142b) und dem Bereich (143a, 143b) zum Unterdrucksetzen des Blutvorhalteteils ausgebildeten Raum (152a, 152b) mit einem oberen Bereich des Blutaufbewahrungsbereichs (110) verbindet.

2. Blutreservoirbehälter mit Blutabgabemechanismus nach Anspruch 1, bei dem das Blutvorhalteteil (103a, 103b) eine Entlüftungsöffnung aufweist, die mit einem oberen Ende des Blutvorhaltebereichs (130a, 130b) verbunden ist.

3. Blutreservoirbehälter (200) mit Blutabgabemechanismus, aufweisend:
einen Blutaufbewahrungsbereich (210);
ein Blutvorhalteteil (203), das einen Blutvorhaltebereich enthält, der mit dem Blutaufbewahrungsbereich verbunden ist, wobei der Blutvorhaltebereich eine Menge an Blut vorhält, die proportional zu der Menge des in dem Blutaufbewahrungsbereich aufbewahrten Blutes ist, wenn die Menge des in dem Blutaufbewahrungsbereich (210) aufbewahrten Blutes gleich oder kleiner einem vorbestimmten Wert wird, wobei es der Blutvorhaltebereich erlaubt, dass Blut aus seinem Innenraum ausströmt, wenn von außen unter Druck gesetzt; und
einen Bereich (204) zum Unterdrucksetzen des Blutvorhalteteils, der arbeitet, wenn Blut aus dem Blutvorhalteteil ausgetrieben werden soll, **gekennzeichnet durch**
die Tatsache, dass das Blutvorhalteteil ein aus einem weichen Material gefertigter röhrenförmiger Körper (203) ist,
die Tatsache, dass der Bereich zum Unterdrucksetzen des Blutvorhalteteils eine Pumpe (204) ist, die den aus einem weichen Material gefertigten röhrenförmigen Körper zusammendrückt, um Blut aus einem Innenraum des röhrenförmigen Körpers herauszudrängen,
einen Hüllenbereich (211), der den aus einem weichen Material gefertigten röhrenförmigen Körper (203) und einen Pumpabschnitt, d. h. einen Bereich, der den aus einem weichen Material gefertigten röhrenförmigen Körper zusammendrückt, um Blut aus dem Innenraum des röhrenförmigen Körpers herauszudrängen, luftdicht von der Umgebung trennt; und
einen Verbindungsweg (185), der einen Innenraum des Hüllenbereichs (211) mit einem oberen Bereich des Blutaufbewahrungsbereichs (210) verbindet.

## Revendications

1. Dispositif réservoir de sang (100 ; 150) équipé d'un mécanisme de distribution de sang, le dispositif comprenant :
une partie stockage de sang (110) ;
un élément de réserve de sang (103a, 103b) comportant une partie réserve de sang (130a, 130b) qui communique avec la partie stockage de sang, la partie réserve de sang contenant une quantité de sang proportionnelle à une quantité de sang stockée dans la partie stockage de sang lorsque la quantité de sang stockée dans la partie stockage de sang (110) devient inférieure ou égale à une valeur prédéterminée, la partie réserve de sang laissant le sang s'écouler à l'extérieur lorsqu'elle est pressée depuis l'extérieur ; et
une unité d'entraînement de distribution de sang (104a, 104b) qui fonctionne lorsque du sang doit être envoyé depuis l'intérieur de la partie réserve de sang,
l'élément de réserve de sang comportant une partie corps (141a, 141b) de l'élément de réserve de sang, laquelle partie corps est faite d'un matériau dur, et une membrane souple (142a, 142b) dont la partie périphérique est retenue par la partie corps de l'élément de réserve de sang,
l'unité d'entraînement de distribution de sang (104a, 104b) comportant une partie de mise en pression (143a, 143b) d'élément de réserve de sang, faite d'un matériau plat dilatable, et un orifice d'écoulement de distribution de sang (144a, 144b) pour dilater et contracter la partie de mise en pression d'élément de réserve de sang,
**caractérisé par** un passage communicant (185a, 185b) qui relie un espace (152a, 152b) formé entre la membrane (142a, 142b) et la partie de mise en pression (143a, 143b) d'élément de réserve de sang à une partie supérieure de la partie stockage de sang (110).

2. Dispositif réservoir de sang équipé d'un mécanisme de distribution de sang selon la revendication 1, dans lequel l'élément de réserve de sang (103a, 103b) comprend un orifice de prise d'air qui communique avec une extrémité supérieure de la partie réserve de sang (130a, 130b).

3. Dispositif réservoir de sang (200) équipé d'un mécanisme de distribution de sang comprenant :
une partie stockage de sang (210) ;
un élément de réserve de sang (203) comportant une partie réserve de sang qui communique avec la partie stockage de sang, la partie réserve de sang contenant une quantité de sang proportionnelle à une quantité de sang stockée dans la partie stockage de sang lorsque la quantité de sang stockée dans la partie stockage de sang (210) devient inférieure ou égale à une valeur prédéterminée, la partie réserve de sang laissant le sang s'écouler à l'extérieur lorsqu'elle est pressée depuis l'extérieur ; et
une partie de mise en pression (204) d'élément de réserve de sang qui fonctionne lorsque du sang doit être expulsé de l'élément de réserve de sang, **caractérisé en ce que**
l'élément de réserve de sang est un corps tubulaire (203) fait d'un matériau mou,
la partie de mise en pression d'élément de réserve de sang est une pompe (204) qui comprime le corps tubulaire en matériau mou pour expulser le sang contenu à l'intérieur du corps tubulaire,
un élément de boîtier (211) qui sépare, de manière étanche à l'air par rapport à l'extérieur, le corps tubulaire en matériau mou (203) d'un site de pompe qui est une partie qui comprime le corps tubulaire en matériau mou pour expulser le sang contenu à l'intérieur du corps tubulaire ; et
un passage communicant (185) qui relie un espace intérieur de l'élément de boîtier (211) à une partie supérieure de la partie stockage de sang (210).
